# EUROPÄISCHE PATENTANMELDUNG

(11) **EP 0 522 364 A1**
(43) Veröffentlichungstag der Anmeldung: **13.01.1993**
(21) Anmeldenummer: 92110761.1
(22) Anmeldetag: 26.06.1992
(51) Int. Cl.: C07D 251/08, C08G 59/50

(54) **2-Cyaniminotetrahydro--1H-1, 3,5-triazine**

(30) Priorität: 27.06.1991 DE 4121257
(71) Anmelder: SKW Trostberg Aktiengesellschaft, D-83308 Trostberg (DE)
(72) Erfinder: Güthner, Thomas, Dr., W-8223 Trostberg (DE)
(74) Vertreter: Huber, Bernhard, Dipl.-Chem.

(57) **Zusammenfassung**

Neue 2-Cyaniminotetrahydro-1H-1,3,5-triazine der allgemeinen Formel (I), 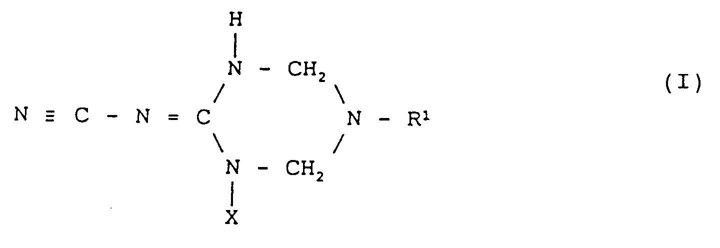
worin R¹ ein Wasserstoffatom, eine Cyanogruppe, einen primären oder sekundären ggf. ungesättigten aliphatischen, cycloaliphatischen oder araliphatischen substitutierten oder unsubstituierten Kohlenwasserstoffrest mit 1 bis 12 Kohlenstoffatomen und X ein Wasserstoffatom oder eine -CH₂-N(R² )₂-Gruppe bedeutet und R² für einen aliphatischen, cycloaliphatischen oder aromatischen Kohlenwasserstoffrest mit 1 bis 10 C-Atomen oder N(R² )₂ für einen stickstoffhaltigen heterocyclischen Rest steht, wobei R¹ auch ein bi- oder mehrfunktioneller Rest, der mehrere Einheiten I verknüpft, sein kann.

## Beschreibung

Gegenstand der vorliegenden Erfindung sind 2-Cyaniminotetrahydro-1H-1,3,5-triazine, Verfahren zu ihrer Herstellung sowie ihre Anwendung bei der Härtung von Epoxidharzen.

Dicyandiamid ist ein Härtungsmittel für Epoxidharze mit breitem Anwendungsbereich. Trotz seiner Vorteile, wie lange Lagerfähigkeit der daraus zubereiteten wärmehärtbaren Mischungen, hohe Festigkeit der gehärteten Mischungen, günstiger Preis und niedrige Einsatzmengen, hat Dicyandiamid gewisse Nachteile, die seine Anwendung in der Technik erschweren. Zu nennen sind hier die geringe Reaktivität gegenüber Epoxidharzen, die geringe Löslichkeit im Harzsystem, die in vielen Fällen die Anwendung toxikologisch problematischer Lösemittel wie Methoxyethanol oder Dimethylformamid notwendig macht, sowie die geringe Feuchtigkeits- und Wasserbeständigkeit der daraus hergestellten gehärteten Mischungen.

Der vorliegenden Erfindung lag daher die Aufgabe zugrunde, neue chemische Verbindungen bereitzustellen, welche die genannten Nachteile des Dicyandiamids nicht aufweisen, sondern gute Härtungseigenschaften verbunden mit besseren anwendungstechnischen Eigenschaften der mit Hilfe dieser Verbindungen gehärteten Epoxidharze aufweisen.

Diese Aufgabe wurde erfindungsgemäß durch 2-Cyaniminotetrahydro-1H-1,3,5-triazine der allgemeinen Formel (I), 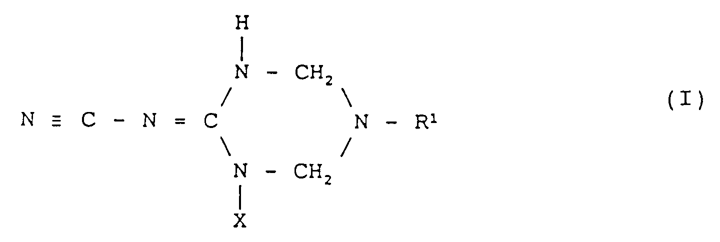
worin R¹ ein Wasserstoffatom, eine Cyanogruppe, einen primären oder sekundären ggf. ungesättigten aliphatischen, cycloaliphatischen oder araliphatischen substitutierten oder unsubstituierten Kohlenwasserstoffrest mit 1 bis 12 Kohlenstoffatomen und X ein Wasserstoffatom oder eine -CH₂-N(R² )₂-Gruppe bedeutet und R² für einen aliphatischen, cycloaliphatischen oder aromatischen Kohlenwasserstoffrest mit 1 bis 10 C-Atomen oder N(R² )₂ für einen stickstoffhaltigen heterocyclischen Rest steht, wobei R¹ auch ein bi- oder mehrfunktioneller Rest, der mehrere Einheiten I verknüpft, sein kann.

Die erfindungsgemäßen Verbindungen der allgemeinen Formel (I) sind neu. Sie besitzen überraschenderweise eine gute Löslichkeit in gebräuchlichen Lösemitteln. Außerdem weisen die mit den erfindungsgemäßen Verbindungen gehärteten Epoxidharzmischungen eine deutlich erhöhte Wärmebeständigkeit sowie Wasser- und Feuchtigkeitsbeständigkeit im Vergleich zu den mit Dicyandiamid gehärteten Harzen auf, was ebenfalls nicht vorhersehbar war.

Die aliphatischen Kohlenwasserstoffreste können linear oder verzweigt sowie gesättigt oder ungesättigt sein. Die Kohlenwasserstoffreste können zusätzlich noch weitere Substituenten tragen, wie z.B. -OH, -OR³ (R³ = Alkyl-, Phenyl-), -N(R⁵ )₂ (R⁵ = H, Alkyl-) oder -CN.

Bevorzugt sind 2-Cyaniminotetrahydro-1H-1,3,5-triazine der allgemeinen Formel (I), worin R¹ ein Wasserstoffatom, eine Cyanogruppe, einen aliphatischen Rest mit 1 bis 8 Kohlenstoffatomen, einen cycloaliphatischen Rest mit 5 bis 10 Kohlenstoffatomen oder einen araliphatischen Rest mit 7 bis 12 Kohlenstoffatomen bedeutet und X = H ist.

R¹ kann auch ein zwei- oder mehrfunktioneller Rest sein, der mehrere Einheiten (I) miteinander verknüpft. Beispiele für solche Reste sind -(CH₂)ₙ-Brücken mit n = 2 bis 10, die gegebenenfalls noch substituiert oder verethert sein können.

Ebenfalls bevorzugt sind 2-Cyaniminotetrahydro-1H-1,3,5-triazine der Formel (I), in denen X eine -CH₂-N(R²)₂-Gruppe ist, in der R² für einen aliphatischen Kohlenwasserstoffrest mit 1 bis 4 Kohlenstoffatomen, einen cycloaliphatischen Kohlenwasserstoffrest mit S bis 10 Kohlenstoffatomen oder einen aromatischen Kohlenwasserstoffrest mit 6 bis 10 Kohlenstoffatomen steht.

Wenn der Rest -N(R² )₂ einen stickstoffhaltigen heterocyclischen Rest bedeutet, werden Fünfringe, wie Pyrrol-, Imidazol oder Benzimidazol, oder Sechsringe, wie Morpholin oder Piperidin, bevorzugt. Diese Heterocyclen können ebenfalls substituiert oder unsubstituiert sein.

Die erfindungsgemäßen 2-Cyaniminotetrahydro-1H-1,3,5-triazine lassen sich technisch relativ einfach durch Umsetzung von Dicyandiamid mit Formaldehyd und einem primären Amin in wäßrigem Medium herstellen, wobei sich an diese Umsetzung gegebenenfalls noch eine zweite Reaktionsstufe mit Formaldehyd und einem sekundären Amin anschließen kann.

Als primäre Amine R¹-NH₂ können sowohl mono- als auch polyfunktionelle primäre Amine eingesetzt werden.

Nachfolgend sind Beispiele für monofunktionelle primäre Amine, die zur Herstellung der erfindungsgemäßen Verbindungen (I) bevorzugt sind, aufgeführt: 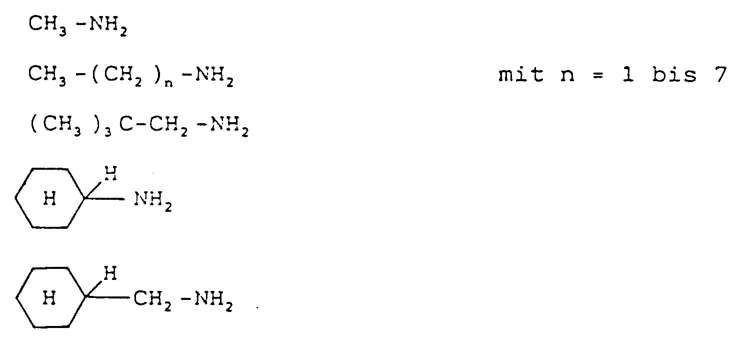 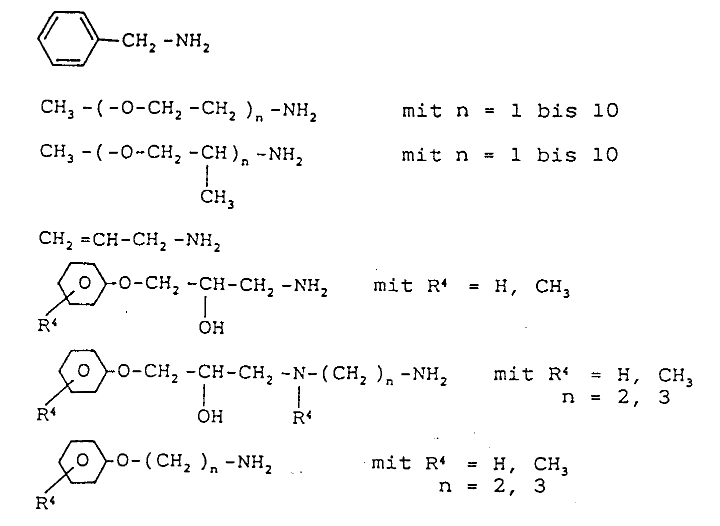
Beispiele für bevorzugte polyfunktionelle primäre Amine sind: 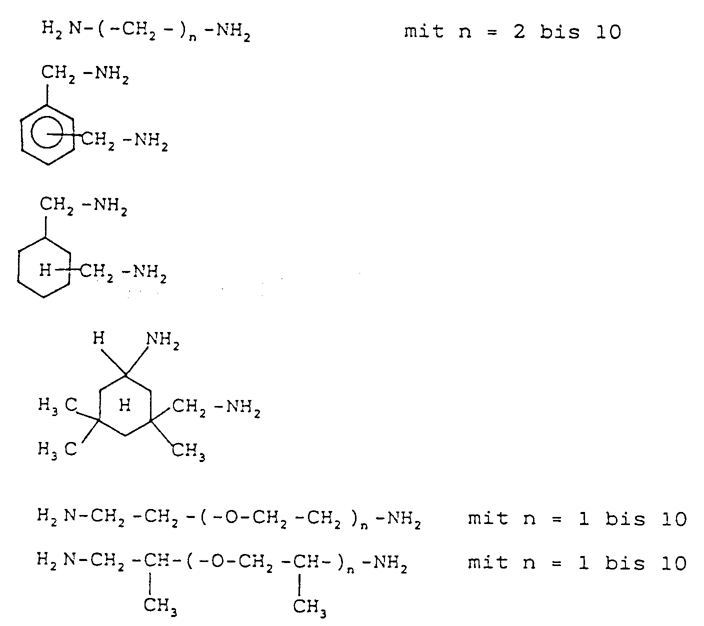 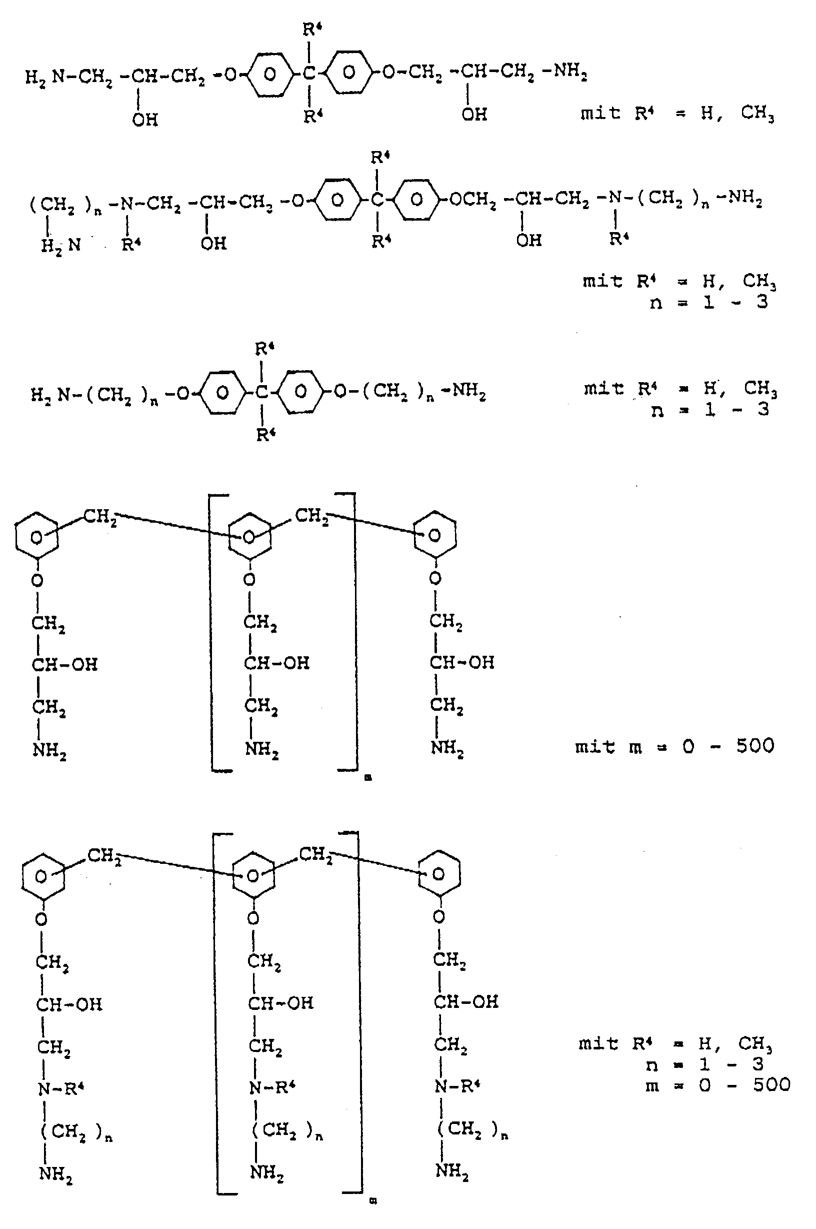 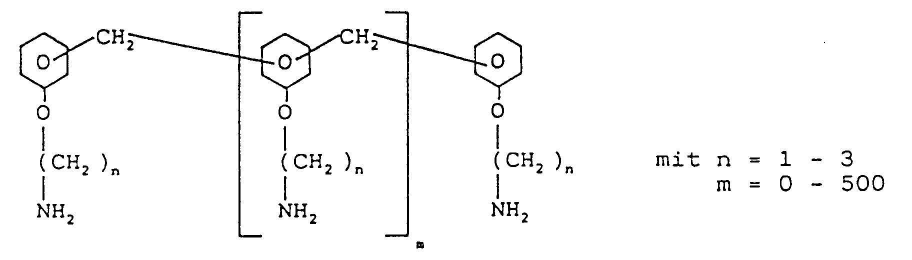
Mit Hilfe dieser polyfunktionellen primären Amine lassen sich mehrere 2-Cyaniminotetrahydro-1H-1,3,5-triazin-5-yl-Reste über R¹ miteinander verknüpfen.

Die bevorzugten 2-Cyaniminotetrahydro-1H-1,3,5-triazine gemäß Anspruch 3 lassen sich ebenfalls sehr einfach aus den Verbindungen von Anspruch 2 durch Umsetzung mit Formaldehyd und einem sekundären Amin herstellen.

Beispiele für geeignete derartige sekundäre Amine sind: 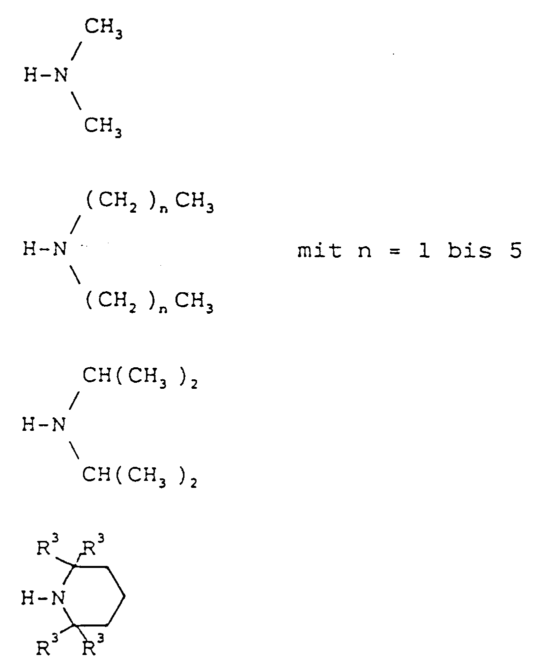 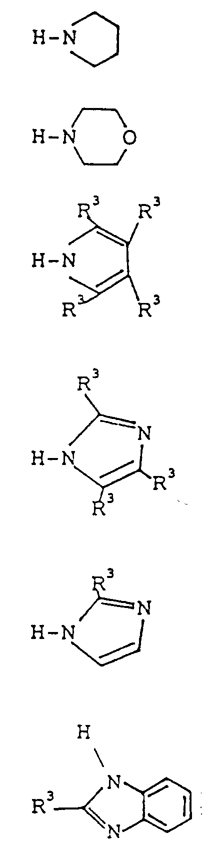
wobei R³ für Wasserstoff, einen aliphatischen Rest mit 1 bis 4 C-Atomen oder einen Phenylrest steht.

Zur Herstellung der erfindungsgemäßen Verbindungen wird Dicyandiamid mit Formaldehyd und einem primären Amin H₂N-R¹ vorzugsweise in wäßriger Lösung umgesetzt.

Die Temperatur kann in weiten Bereichen variiert werden, vorzugsweise liegt sie zwischen 20 und 110°C. Der Zeitbedarf zum Abschluß der Umsetzung liegt zwischen 0,2 und 8 Stunden. Gegebenenfalls kann zur Erzielung eines speziellen Produkts der pH-Wert der Reaktionslösung auf einen bestimmten Wert zwischen 6 und 12 eingestellt werden.

Die Reihenfolge der Zugabe der einzelnen Komponenten ist unkritisch, zur Erzielung spezieller Produkteigenschaften kann jede Komponente auch in mehreren Schritten zugegeben werden.

Die einzusetzenden molaren Mengen an Formaldehyd und primärem Amin, bezogen auf die eingesetzte molare Menge Dicyandiamid, können in weiten Bereichen variiert werden.

Vorzugsweise wird das primäre Amin H₂N-R¹ im Molverhältnis 0,8 bis 1,2, besonders bevorzugt 0,9 bis 1,1, bezogen auf Dicyandiamid eingesetzt. Dabei kann das primäre Amin als Reinsubstanz oder als wäßrige Lösung zur Anwendung kommen.

Formaldehyd wird vorzugsweise im Molverhältnis 1,8 bis 2,6, insbesondere 2,0 bis 2,2, bezogen auf Dicyandiamid, eingesetzt.

Die Umsetzung der erfindungsgemäßen Verbindungen von Formel (I) mit X = H mit weiterem Formaldehyd und einem sekundären Amin H-N(R² )₂ zu solchen Verbindungen der allgemeinen Formel (I) mit X = -CH₂-N(R² )₂ erfolgt bevorzugt in wäßriger Lösung. Die Ausgangsprodukte gemäß Formel (I) mit X = H können entweder in isolierter Form eingesetzt werden, oder aber es wird die rohe Reaktionslösung aus ihrer Synthese direkt weiterverarbeitet.

Die Herstellung der erfindungsgemäßen Verbindungen mit X = -CH₂-N(R²)₂ erfolgt vorzugsweise bei Temperaturen von 50 und 95°C, wobei die Reaktion nach 0,2 bis 8 Stunden beendet ist. Gegebenenfalls kann der pH-Wert der Reaktionslösung auf einen bestimmten Wert zwischen 6 und 12 eingestellt werden.

Die Reihenfolge der Zugabe der einzelnen Komponenten ist beliebig; zur Erzielung spezieller Produkteigenschaften kann jede Komponente auch in mehreren Schritten zugegeben werden.

Die einzusetzenden molaren Mengen an Formaldehyd und sekundärem Amin, bezogen auf die eingesetzte molare Menge (I)mit X = H, sind in weiten Grenzen variierbar. Vorzugsweise wird das sekundäre Amin H-N(R² )₂ im Molverhältnis 0,5 bis 1,5, insbesondere 0,8 bis 1,2, bezogen auf (I) mit X = H, eingesetzt.

Formaldehyd wird bevorzugt im Molverhältnis 0,9 bis 1,2, insbesondere 1,0 bis 1,1, bezogen auf das eingesetzte sekundäre Amin H-N(R² )₂, verwendet.

Die Isolierung der Produkte aus der Reaktionslösung kann erfolgen entweder durch teilweises Einengen der Reaktionslösung und Abfiltrieren des ausgefallenen Produkts nach dem Abkühlen, oder durch komplettes Einengen der Reaktionslösung.

Bevorzugt ist die letztere Variante, da hierbei ohne wesentliche Nebenprodukte Ausbeuten bis 100 % erzielt werden können.

Die Produkte werden anschließend, gegebenenfalls unter Anwendung erhöhter Temperatur und/oder erniedrigtem Druck, getrocknet.

Die erfindungsgemäßen Verbindungen können flüssig, harzartig oder fest sein. Im Falle von festen Produkten wird eine anschließende Feinmahlung bevorzugt.

Die erfindungsgemäßen Verbindungen eignen sich gut als Härter für hitzehärtbare Epoxidharzmassen. Als Epoxidharze kommen dabei alle handelsüblichen Produkte in Frage, die üblicherweise mehr als eine 1,2-Epoxidgruppe aufweisen und gesättigt oder ungesättigt, aliphatisch, cycloaliphatisch, aromatisch oder heterocyclisch sein können und eventuell Substituenten wie Halogene, Phosphor, Hydroxylgruppen etc. enthalten.

Besonders bevorzugt finden Epoxidharze auf Basis der Polyglycidylether von Bisphenol A, Bisphenol F, Bisphenol S oder Tetrabrombisphenol A oder aber Glycidylether von NovolakHarzen Verwendung.

Die erfindungsgemäßen Härter können in Anteilen von 1 bis 30 Gew.-%, bezogen auf das Epoxidharz, eingesetzt werden. Abhängig von Art und Molekulargewicht des verwendeten Harzes sind 3 bis 15 Gew.-% besonders geeignet.

Die erfindungsgemäßen Härter finden mit oder ohne Zusatz eines bekannten Beschleunigers Verwendung. So können als Beschleuniger tertiäre Amine (z.B. Benzyldimethylamin), Imidazole (z.B. 1H-Imidazol, 2-Methyl-1H-imidazol, 2-Ethyl-4-methyl-1H-imidazol) oder substituierte Harnstoffderivate (z.B. N,N-Dimethyl-N'-phenylharnstoff, N,N-Dimethyl-N'-(4-Chlorphenyl)harnstoff, 2,4-Bis(N,N-dimethylureido)toluol) eingesetzt werden. Die verwendeten Zusatzmengen des Beschleunigers können in weiten Bereichen schwanken, vorzugsweise betragen sie 0,05 bis 5 Gew.-%, bezogen auf das eingesetzte Harz.

Die Härtung der Epoxidharze mit den erfindungsgemäßen Härtern erfolgt vorzugsweise bei Temperaturen von 80 bis 200°C, besonders bevorzugt bei 100 bis 180°C.

Die Gelierzeiten liegen je nach Art und Menge des Härters und des Beschleunigers typischerweise bei 1 bis 10 Minuten.

Die unter Zusatz der erfindungsgemäßen Härter hergestellten härtbaren Mischungen sind bei Raumtemperatur mehrere Tage lagerfähig.

Vorteilhaft für die Anwendung der erfindungsgemäßen Verbindungen sind die hervorragenden Eigenschaften der daraus hergestellten gehärteten Mischungen. Gegenüber der Verwendung von Dicyandiamid ist eine deutlich erhöhte Wärmebeständigkeit sowie eine erhöhte Wasser- und Feuchtigkeitsbeständigkeit zu verzeichnen.

Für die Anwendung günstig erweist sich die gegenüber Dicyandiamid erhöhte Löslichkeit in geeigneten Lösemitteln.

Ein weiterer Vorteil der erfindungsgemäßen Verbindungen ist ihre einfache und preisgünstige Herstellung aus leicht erhältlichen Ausgangsstoffen, die eine technische Anwendung besonders günstig erscheinen läßt.

Die folgenden Beispiele sollen die Erfindung näher erläutern.

### Beispiele

### Beispiel 1

### 2-Cyanimino-5-methyltetrahydro-1H-1,3,5-triazin

81 g Formaldehydlösung (37 %ig), 100 ml Wasser und 42 g Dicyandiamid werden 30 Minuten bei Raumtemperatur gerührt, anschließend werden 38,8 g Methylamin-Lösung (40 %ig) zugegeben. Die Mischung wird 2 Stunden bei 70°C gehalten, die erhaltene Lösung wird sofort zur Trockene eingedampft und das weiße kristalline Produkt getrocknet.

| | |
|---|---|
| Ausbeute: | 67 g (96 %) |
| Elementaranalyse: | gef.: C 42,50, H 6,52, N 50,90 % |
| | ber.: C 43,17, H 6,48, N 50,30 % |
| IR-Spektrum: | intensive Banden bei 2152 und 2171 cm⁻¹ |
| Dicyandiamid-Gehalt: | < 0,5 % |

### Beispiel 2

### 5-Butyl-2-cyaniminotetrahydro-1H-1,3,5-triazin

43,0 g Dicyandiamid, 37 g n-Butylamin, 108 ml Formaldehydlösung (30 %ig) und 400 ml Wasser werden vermischt. Die Temperatur der Lösung steigt auf 54°C und wird anschließend 1 Stunde bei 60°C gehalten. Aus der erhaltenen Lösung werden 300 ml Wasser abdestilliert, die verbleibende Lösung wird auf 4°C abgekühlt und das ausgefallene Produkt abgesaugt und getrocknet.

| | |
|---|---|
| Ausbeute: | 74,2 (82 %) |
| Elementaranalyse: | gef.: C 52,10, H 8,67, N 36,78 % |
| | ber.: C 53,02, H 8,34, N 38,64 % |
| IR-Spektrum: | intensive Bande bei 2165 cm⁻¹ |
| Dicyandiamid-Gehalt: | < 0,5 % |

### Beispiel 3

### 1,2-Bis(2-cyaniminotetrahydro-1H-1,3,5-triazin-5-yl)-ethan

14,5 g Ethylendiamin, 100 ml Wasser und 100 ml Formaldehydlösung (30 %ig) werden 0,5 Stunden gerührt. Anschließend werden 42 g Dicyandiamid zugegeben und die Mischungen 2 Stunden auf 70°C erhitzt. Daraufhin wird das Lösungsmittel abgedampft und das Produkt getrocknet.

| | |
|---|---|
| Ausbeute: | 68 g (99 %) |
| Elementaranalyse: | gef.: C 42,39, H 5,83, N 50,77 % |
| | ber.: C 43,48, H 5,80, N 50,72 % |

### Beispiel 4

### 5-Benzyl-2-cyaniminotetrahydro-1H-1,3,5-triazin

42 g Dicyandiamid werden mit 81 g Formaldehydlösung (37 %ig) während 1 Stunde bei 70°C gerührt. Nach Zugabe von 53,5 g Benzylamin wird weitere 2 Stunden bei 70°C gerührt. Die Lösung wird abgekühlt, das ausgefallene Produkt abgesaugt und getrocknet.

| | |
|---|---|
| Ausbeute: | 99,7 g (93 %) |
| Elementaranalyse: | gef.: C 61,77, H 6,21, N 32,14 % |
| | ber.: C 61,38, H 6,09, N 32,53 % |

### Beispiel 5

### 2-Cyanimino-5-(2-methoxyethyl)tetrahydro-1H-1,3,5-triazin

42 g Dicyandiamid werden mit 81 g Formaldehydlösung (37 %ig) 1 Stunde bei 70°C gerührt, 37,5 g 2-Methoxyethylamin werden zugegeben und weitere 2 Stunden bei 70°C gerührt. Die erhaltene Lösung wird zu einer hochviskosen Schmelze eingedampft. Beim Trocknen im Vakuum erstarrt das Produkt zu einer glasartigen spröden Masse.

| | |
|---|---|
| Ausbeute: | 76,2 g (83 %) |
| Elementaranalyse: | gef.: C 45,18, H 7,25, N 38,20 % |
| | ber.: C 45,90, H 7,10, N 38,25 % |

### Beispiel 6

### 2-Cyanimino-1-(N,N-dimethylaminomethyl)-5-methyltetrahydro-1H-1,3,5-triazin

69,5 g eines Produktes nach Beispiel 1 werden bei 40°C in 100 ml Wasser gelöst. Zu dieser Lösung werden 40,5 g Formaldehydlösung (37 %ig) und 37,5 g Dimethylamin (60 %ig) zugegeben und 2 Stunden bei 70°C gerührt. Das Lösungsmittel wird abgedampft und das erhaltene Produkt getrocknet.

| | |
|---|---|
| Ausbeute: | 95 g (97 %) |
| Elementaranalyse: | gef.: C 44,56, H 7,02, N 44,28 % |
| | ber.: C 48,97, H 8,16, N 42,86 % |
| Dicyandiamid-Gehalt: | < 0,2 % |

### Beispiel 7

### 2-Cyanimino-1-(2-methyl-1H-imidazol-1-ylmethyl)-5-methyltetrahydro-1H-1,3,5-triazin

42 g Dicyandiamid, 50 g Wasser, 81,0 g Formaldehydlösung (37 %ig) und 38,8 g Methylamin (40 %ig) werden zusammen 2 Stunden auf 70°C erhitzt. Anschließend werden 40,5 g Formaldehyd (37 %ig) und 41,0 g 2-Methylimidazol zugegeben und weitere 2 Stunden bei 70°C gerührt. Das Lösungsmittel wird abgedampft und das erhaltene Produkt getrocknet.

| | |
|---|---|
| Ausbeute: | 76 g (65 %) |
| Elementaranalyse: | gef.: C 47,58, H 7,79, N 40,45 % |
| | ber.: C 51,50, H 6,44, N 42,06 % |
| Dicyandiamid-Gehalt: | < 0,2 % |

### Beispiel 8

### Härtung von Epoxidharzen

Es wird die härtende Wirkung der erfindungsgemäßen Verbindungen auf epoxidgruppenhaltige Verbindungen untersucht. Folgende Mischungen wurden hergestellt:

| | Epikote 828 Gew.-Teile | Härter Gew.-Teile | Beschleuniger Gew.-Teile |
|---|---|---|---|
| a) | 100 | 10 (Beispiel 1) | - |
| b) | 100 | 10 (Beispiel 1) | 2 2-Methylimidazol |
| c) | 100 | 10 (Beispiel 1) | 2 Benzyldimethylamin |
| d) | 100 | 10 (Beispiel 1) | 2 N,N-Dimethyl-N'-phenylharnstoff |
| e) | 100 | 10 (Beispiel 2) | 2 2-Methylimidazol |
| f) | 100 | 10 (Beispiel 3) | 2 2-Methylimidazol |
| g) | 100 | 10 (Beispiel 4) | 2 2-Methylimidazol |
| h) | 100 | 10 (Beispiel 5) | 2 2-Methylimidazol |
| i) | 100 | 10 (Beispiel 6) | 2 2-Methylimidazol |
| k) | 100 | 10 (Beispiel 7) | - |
| l) | 100 | 7 Dicyandiamid | - (Vergleich) |
| m) | 100 | 7 Dicyandiamid | 2 2-Methylimidazol (Vergleich) |

Von den Mischungen a) bis m) wurden Gelierzeiten bei verschiedenen Temperaturen sowie differenzwärmestrom-kalorimetrische Daten (DDWK nach DIN 51005) ermittelt. Die Heizrate bei letzteren Messungen betrug 10 K/min.

| | Gelierzeit 160°C | Gelierzeit 140°C | DDWK onset °C | DDWK peak °C |
|---|---|---|---|---|
| a) | 2'48'' | 7'30'' | 130,0 | 143,5 |
| b) | 0'57'' | 1'28'' | 104,3 | 130,7 |
| c) | - | - | 82,0 | 118,3 |
| d) | - | - | 129,5 | 144,5 |
| e) | 1'08'' | 1'48'' | 107,9 | 141,3 |
| f) | 1'20'' | 1'38'' | 106,7 | 130,6 |
| g) | 1'01'' | 1'20'' | 112,2 | 135,5 |
| h) | 0'54'' | 1'20'' | 103,0 | 129,1 |
| i) | 1'02'' | 1'16'' | 101,8 | 131,9 |
| k) | 1'14'' | 1'58'' | 108,0 | 137,0 |
| l) | > 30' | > 30' | 191,6 | 200,6 (Vergleich) |
| m) | 0'52'' | 1'12'' | 137,5 | 158,0 (Vergleich) |

### Beispiel 9

### Wasserbeständigkeit

Je 5 x 2 g der Mischungen b) und m) aus Beispiel 8 wurden 30 Minuten bei 120°C und anschließend 1 Stunde bei 180°C zu tablettenförmigen Probekörpern ausgehärtet. Die Glasübergangstemperatur (Tg(1)) der gehärteten Mischung wurde durch DDWK-Messungen ermittelt.

Anschließend wurden die Probekörper 7 Tage bei 80°C in H₂O-Immersion gelagert. Neben der Gewichtszunahme wurden direkt der Tg-Wert (Tg(2)) sowie der Tg-Wert nach 10 Minuten Rekonditionierung bei 180°C (Tg(3)) gemessen. Folgende Meßwerte wurden erhalten:

| Mischung | b) (erfindungsgemäß) | m) (Vergleich) |
|---|---|---|
| Tg (1) | 124,5°C | 125,0°C |
| Gewichtszunahme | 6,6 % | 7,2 % |
| Tg (2) | 108,5°C | 70,0°C |
| Tg (3) | 127,7°C | 107,3°C |

## Patentansprüche

1. 2-Cyaniminotetrahydro-1H-1,3,5-triazine der allgemeinen Formel (I), 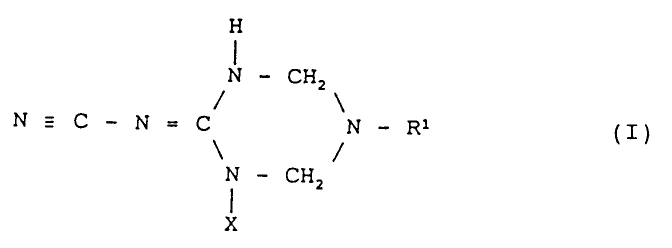 worin R¹ ein Wasserstoffatom, eine Cyanogruppe, einen primären oder sekundären ggf. ungesättigten aliphatischen, cycloaliphatischen oder araliphatischen substitutierten oder unsubstituierten Kohlenwasserstoffrest mit 1 bis 12 Kohlenstoffatomen und X ein Wasserstoffatom oder eine -CH₂-N(R²)₂-Gruppe bedeutet und R² für einen aliphatischen, cycloaliphatischen oder aromatischen Kohlenwasserstoffrest mit 1 bis 10 C-Atomen oder N(R²)₂ für einen stickstoffhaltigen heterocyclischen Rest steht, wobei R¹ auch ein bi- oder mehrfunktioneller Rest, der mehrere Einheiten I verknüpft, sein kann.

2. 2-Cyaniminotetrahydro-1H-1,3,5-triazine nach Anspruch 1,
**dadurch gekennzeichnet,**
daß R¹ ein aliphatischer Rest mit 1 bis 8 Kohlenstoffatomen, ein cycloaliphatischer Rest mit 5 bis 10 Kohlenstoffatomen oder ein araliphatischer Rest mit 7 bis 12 Kohlenstoffatomen ist.

3. 2-Cyaniminotetrahydro-1H-1,3,5-triazine nach Anspruch 1 oder 2,
**dadurch gekennzeichnet,**
daß R² ein aliphatischer Kohlenwasserstoffrest mit 1 bis 4 Kohlenstoffatomen, ein cycloaliphatischer Kohlenwasserstoffrest mit 5 bis 10 Kohlenstoffatomen, ein aromatischer Kohlenwasserstoffrest mit 6 bis 10 Kohlenstoffatomen oder -N(R²)₂ ein substituierter oder unsubstituierter Morpholin-, Piperidin-, Pyrrol-, Imidazol- oder Benzimidazolrest ist.

4. Verfahren zur Herstellung der 2-Cyaniminotetrahydro-1H-1,3,5-triazine (I) nach den Ansprüchen 1 bis 3,
**dadurch gekennzeichnet,**
daß man Dicyandiamid mit Formaldehyd und einem primären Amin der Formel R¹-NH₂ sowie ggf. anschließend mit Formaldehyd und einem sekundären Amin der Formel (R² )₂NH in wäßrigem Medium umsetzt.

5. Verfahren nach Anspruch 4,
**dadurch gekennzeichnet,**
daß man die Umsetzung bei einer Temperatur von 20 bis 110°C vornimmt.

6. Verfahren nach den Ansprüchen 4 und 5,
**dadurch gekennzeichnet,**
daß man die Umsetzung bei einem pH-Wert von 6 bis 12 durchführt.

7. Verfahren zur Herstellung einer Verbindung nach Anspruch 2,
**dadurch gekennzeichnet,**
daß man das Molverhältnis von primärem Amin zu Dicyandiamid auf 0,8 bis 1,2 und das Molverhältnis Formaldehyd zu Dicyandiamid auf 1,8 bis 2,6 einstellt.

8. Verfahren nach Anspruch 7,
**dadurch gekennzeichnet,**
daß das Molverhältnis von primärem Amin zu Dicyandiamid 0,9 bis 1,1 und das Molverhältnis Formaldehyd zu Dicyandiamid 2,0 bis 2,2 beträgt.

9. Verfahren zur Herstellung einer Verbindung nach Anspruch 3,
**dadurch gekennzeichnet,**
daß man eine Verbindung nach Anspruch 1 oder 2 mit X = Wasserstoff mit Formaldehyd und einem sekundären Amin (R² )₂NH umsetzt.

10. Verfahren nach Anspruch 9,
**dadurch gekennzeichnet,**
daß das Molverhältnis von sekundärem Amin zur Verbindung nach Anspruch 1 oder 2 mit X = Wasserstoff 0,5 bis 1,2, vorzugsweise 0,8 bis 1,2, und das Molverhältnis Formaldehyd zu sekundärem Amin 0,9 bis 1,2, vorzugsweise 1,0 bis 1,1 beträgt.

11. Verwendung der 2-Cyaniminotetrahydro-1H-1,3,5-triazine nach den Ansprüchen 1 bis 3 als Härter für Epoxidharzmassen.

12. Verwendung der 2-Cyaniminotetrahydro-1H-1,3,5-triazine nach Anspruch 11 in einer Menge von 1 bis 30 Gew.-%, bezogen auf das Gewicht des Epoxidharzes.
